# EUROPEAN PATENT SPECIFICATION

(11) **EP 3 946 035 B1**
(45) Date of publication and mention of the grant of the patent: **27.08.2025**
(21) Application number: 20785028.0
(22) Date of filing: 30.03.2020
(51) Int. Cl.: A61B 5/25

(54) **PHYSIOLOGICAL SIGNAL COLLECTION ELECTRODES AND PERFORMANCE MONITORING APPARATUS COMPRISING SAME**
ELEKTRODEN ZUR ERFASSUNG PHYSIOLOGISCHER SIGNALE UND LEISTUNGSÜBERWACHUNGSVORRICHTUNG DAMIT
ÉLECTRODES DE COLLECTE DE SIGNAL PHYSIOLOGIQUE ET APPAREIL DE SURVEILLANCE DE PERFORMANCE LES COMPRENANT

(30) Priority: 29.03.2019 US 201916368895
(43) Date of publication of application: 09.02.2022
(73) Proprietor: Dayton Technologies Limited, New Territories, Hong Kong (HK)
(72) Inventor: YUEN, Paul Anthony, Kwai Chung, New Territories, Hong Kong (HK)
(74) Representative: Murgitroyd & Company
(86) International application number: PCT/IB2020/052995
(87) International publication number: WO 2020/201979

(56) References cited:
- EP-A1- 0 966 917
- CN-A- 102 858 236
- CN-A- 105 286 106
- US-A1- 2005 096 556
- US-A1- 2007 093 707
- US-A1- 2008 177 168
- US-A1- 2013 123 601
- US-A1- 2013 131 460
- US-A1- 2013 158 380
- US-A1- 2019 298 203

## Description

### FIELD

The present disclosure relates to physiological signal collection electrodes and performance monitoring apparatus comprising same.

### BACKGROUND

Physiological signals in the form of bio-electrical signals can be collected by physiological signal collection electrodes for processing, for example, by an electronic device to provide useful information on the physical state of a person wearing the electrodes.

Different types of physiological signal collection electrodes are required for collecting different types of physiological signals and different types of electronic devices are required to receive and/or process the different types of physiological signals.

It would be advantageous if physiological signal collection electrodes and electronic devices can be easily conveniently assembled to form different types of physiological signal collectors.

US2013/158380A1 discloses a biological information detection device having a main body and a biological signal detection portion which are integrally provided through a mechanical connection portion that mechanically interconnects the main body and the biological signal detection portion. The device has an electrical connection portion that electrically interconnects the device main body and the electrode of the biological signal detection portion, but is physically separated therefore.

US2008/0177168A1 discloses an ECG lead set including an ECG electrode assembly and a lead set hub.

### Summary

Physiological signal collection electrodes and performance monitoring apparatus comprising same are disclosed in this disclosure.

The physiological signal collection electrode comprises a signal collection portion, a signal output portion having a signal output end, and a bridging portion interconnecting the signal collection portion and the signal output portion. The signal collection portion, the bridging portion and the signal output portion are distributed in series along a longitudinal axis and the signal output portion is on a longitudinal end of the longitudinal axis. The signal collection portion comprises a signal collection surface for collection of physiological signals from a living body by body contact. The signal output portion comprises a signal output pad having a forward longitudinal end and a signal output terminal at the forward longitudinal end of the signal output pad. The signal output pad has a first surface, a second surface, and a peripheral surface that includes an end surface which is a forward-end surface interconnecting the first surface and the second surface. The signal output terminal is in physical and electrical contact with both the first surface and the second surface, and has a signal output surface which is configured to output signals collected by the signal collection portion and which is an exterior surface that is exposed for making electrical connection and extends about a traverse axis at the longitudinal end of the signal output pad to define a longitudinal end of the electrode, the traverse axis being orthogonal to the longitudinal axis. The signal collection surface is parallel to and offset from the longitudinal axis and offset from the first and second surfaces of the signal output pad. The signal output terminal is attached to the signal output pad and extends along a transverse direction which is orthogonal to the longitudinal axis and has a signal output surface which surrounds the forward-end surface of the signal output pad. The signal output terminal is configured as an elongate C-shaped clamp, the C-shaped clamp having a main body which is a hollow metal bar defining an internal surface and an external surface surrounding the internal surface. The internal surface is in compressive physical abutment with the signal output pad and the external surface is the signal output surface.

In embodiments when the signal output terminal is attached to the signal output pad, the peripheral surface includes a forward-end surface and the signal output terminal has a signal output surface which extends to surround the forward-end surface.

The invention also provides a performance monitoring apparatus comprising an electrode or a plurality of electrodes according to any of the preceding claim and an electronic device connected to the electrode or the plurality of electrodes, wherein the electrode is configured for collecting ECG signals and the electronic device is configured for collecting and processing ECG signals.

### Figures

The disclosure is made by way of example and with reference to the accompanying Figures, in which:
Figures 1A and 1B are perspective views of an example signal collection apparatus according to the disclosure,
Figures 2 is a perspective view of an example signal collection strap sub-assembly according to the disclosure,
Figure 3A and 3B are perspective views of example first and second electrodes of the sub-assembly of Figure 2;
Figure 3C shows an example electrode having a tapered output end,

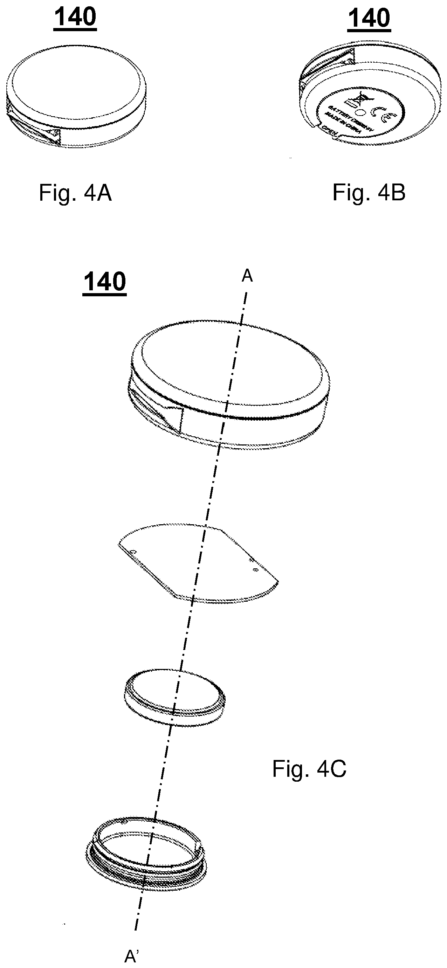

Figures 4A, 4B and 4C are various views of an example electronic apparatus which is attachable to the signal collection apparatus to form an example physiological signal apparatus,
Figures 5 is a perspective views of another sub-assembly comprising a signal collection apparatus and a strap-ends connector of the present disclosure,
Figures 6A1, 6A2, 6A3 and 6A4 are various views of the first signal collection electrode of the assembly of Figure 5,
Figures 6B1, 6B2, 6B3 and 6B4 are various views of the second signal collection electrode of the assembly of Figure 5,
Figures 6C1 and 6C2 are perspective views of an example signal output terminal of the example signal collection apparatus,

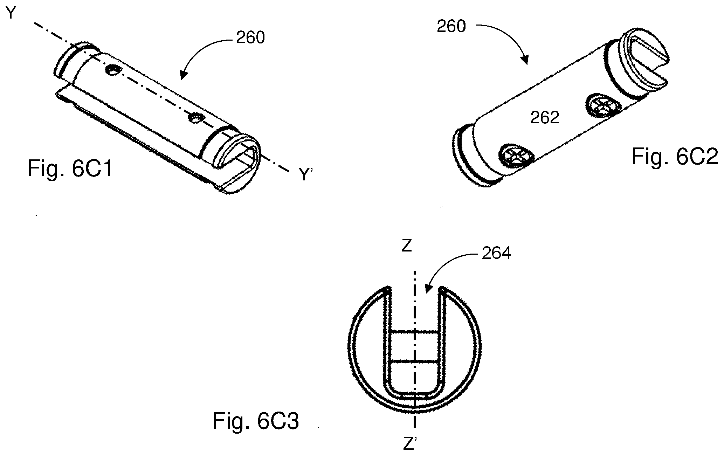

Figure 6C3 is an end view of the signal output terminal viewed from one longitudinal end,

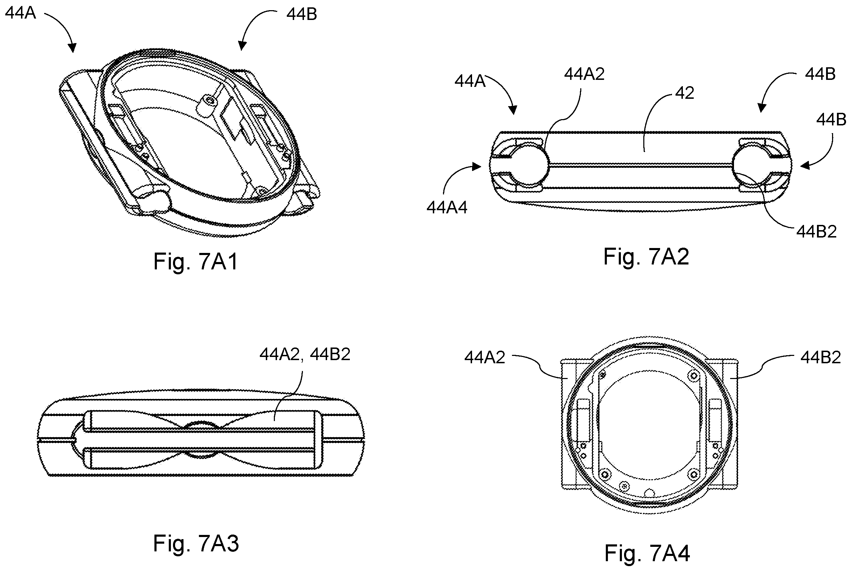

Figures 7A1, 7A2, 7A3 and 7A4 are various views showing a portion of the main housing and the anchoring terminal housings,

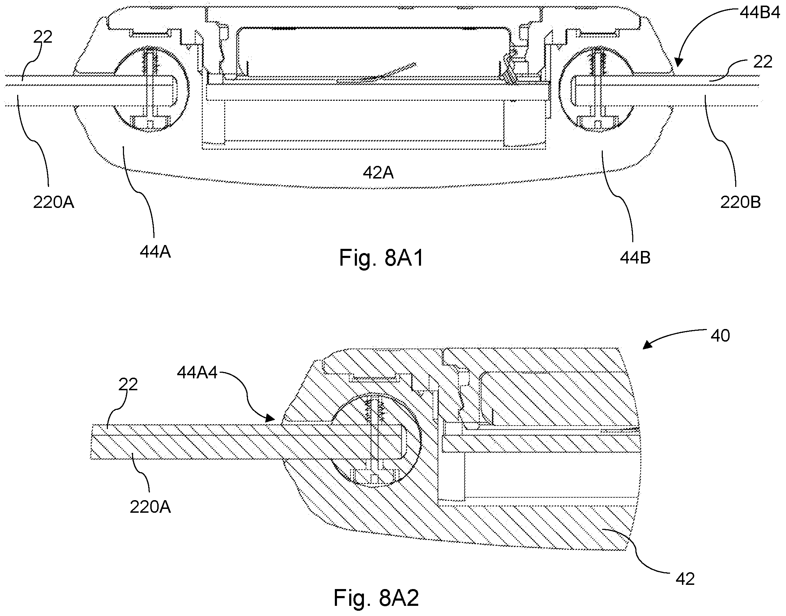

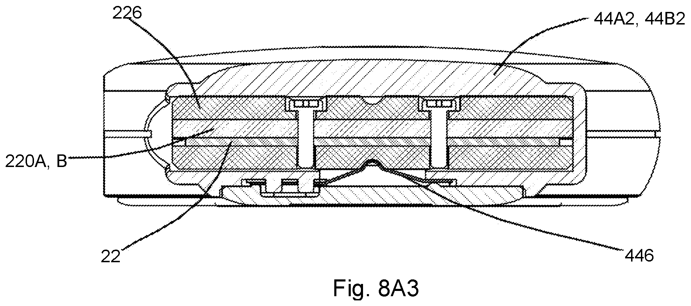

Figure 8A1 is a schematic side view taken along the longitudinal direction of the signal output terminal in the region of the electronic device,
Figure 8A2 is an enlarged cross-sectional view of a portion of Figure 8A1, and
Figure 8A3 is a cross-sectional view showing anchoring relationships between the signal collection apparatus and the main housing of the electronic device taken along the longitudinal direction of the strap.

### Detailed description of embodiments

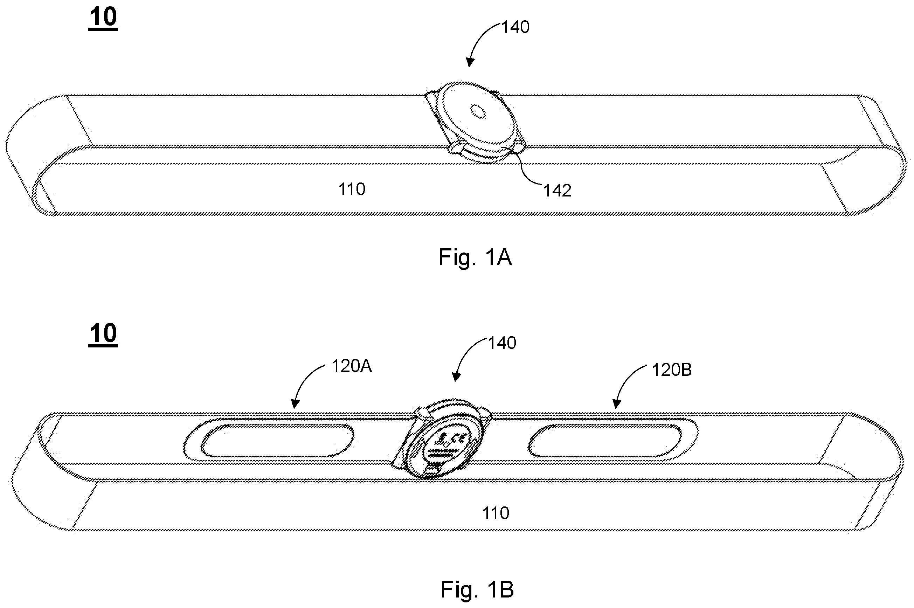

Referring to Figures 1A and 1B, an example physiological signal collection apparatus **10** comprises a carrier **110,** a plurality of physiological signal collection electrodes **120** mounted on the carrier, and an electronic device **140** detachably connected to the electrodes **120.**

The electronic device may be an electronic signal collection device which is configured to receive physiological signals collected by a physiological signal electrode or by the plurality of physiological signal collection electrodes for processing and/or forward transmission.

In example embodiments, the electrodes are disposed on two sides of the electronic signal collection device to collect physiological signals from two lateral sides of a living body.

Each physiological signal collection electrode (or electrode in short) comprises a signal collection portion **122** having a signal collection surface **123,** a signal output portion **124** having a signal output end **125,** a bridging portion **126** interconnecting the signal collection portion and the signal output portion.

The signal collection portion comprises a first surface which is a signal collection surface and a second surface which is a non-signal-collection surface. The first surface is a body contact surface which is configured to be in physical abutment and electrical contact with a body surface during use. When the signal collection surface is in physical abutment and electrical contact with a body surface during use, physiological signals of the living body will travel from the living body to the signal collection surface, move towards the signal output end of the signal output portion, and then collected by the electronic signal collection device. The signal collection surface has a body-facing orientation during use so that physiological signals can be collected from a body surface. The second surface of the signal collection portion has a non-body facing orientation during use, the non-body facing being a facing orientation which is directly opposite to the body-facing orientation. Directly opposite herein means a difference in facing orientation of 180 degrees or about 180 degrees.

The signal collection surface is formed on a salient region on the signal collection portion. The salient region which defines the signal collection surface is formed as a signal collection island and the island is surrounded and/or delineated by a peripheral region, which defines a base portion surrounding the signal collection island. The signal collection surface is formed as an elevated plateau surface which is elevated above the peripheral region to facilitate good electrical contact with the body surface. In example embodiments, the island has a hollow interior and defines a hollow compartment. The signal collection island may be integrally formed of a conductive polymeric material such as carbonized rubber. The signal collection island and the peripheral region may be integrally formed of the same conductive material or different materials. In some embodiments, the peripheral region is formed of a non-conductive material such as a non-conductive elastomer. In some embodiments, the peripheral region is formed of a conductive material such as a conductive elastomer but is electrically insulated from the body surface by an insulating layer such as an insulating layer over-moulded on the peripheral region.

The example electrode is elongate and extends along a center axis. The center axis of the electrode is also a longitudinal axis of the electrode. The signal collection portion, the bridging portion and the signal output portion are distributed in series along the longitudinal axis. The bridging portion is a signal transmission portion which is to relay electrical signals between the signal collection portion and the signal output portion. In some embodiments, the bridging portion is a narrowed portion intermediate the signal collection portion and the signal output portion. In example embodiments, the electrode is symmetrical about the center axis.

The signal collection surface may have different shapes, for example, circular, oval, polygonal such as square, rectangular or other regular polygonal shapes. Where the signal collection surface has a polygonal shape, the corners may be rounded to promote user comfort.

In example embodiments, the signal collection surface is elongate and has a major axis which is parallel to the longitudinal axis and defines its length, a minor axis which is orthogonal to the longitudinal axis and defines its width, and a signal collection area which is characterized by its width and its length. In general, the length of the signal collection surface is measured in an axial direction which is parallel to the longitudinal axis, and the width of the signal collection surfacing is measured in a direction which is orthogonal to the axial direction. The signal collection island, and therefore the signal collection surface, has rounded corners to promote enhanced user comfort. In example embodiments, the peripheral portion has rounded corners to promote wearing comfort
In example embodiments such as the present, the example signal collection portion is singly formed as an elastomeric signal collection pad. A pad herein means a form factor in which the thickness is substantially smaller than the width or length (whichever is smaller). The thickness of the signal collection portion herein means the separation, or average separation, between the first surface and second surface. The term substantially smaller herein means a ratio of 20%, 15%, 10% or less. In example embodiments, the signal collection surface has a width of between 1cm and 2.5cm, or a range or ranges selected from any combination of 1.5cm, 2cm, 2.5cm. In example embodiments, the signal collection surface has a length of between 5cm and 10cm, or a range or ranges selected from any combination of 6cm, 7cm, 8cm, 9cm, 10cm. The width and length may be selected or adjusted according to age, gender, skin conditions of a user.

In this example, the signal collection surface has a signal collection area of approximately 15 square centimetres (cm²), measuring 8cm (length) and 1.9cm (width) with rounded longitudinal ends. In general, a signal collection surface of between 9-25 square centimetres (cm²) would be beneficial.

The signal output portion has a first surface, a second surface having a directly opposite facing orientation to the first surface, and a third surface which is a peripheral surface interconnecting the first surface and the second surface. The peripheral surface includes an end surface which is on a signal output end of the signal output portion. The signal output end of the signal output portion is on a longitudinal end of the electrode and is a distal end which is distal from the signal collection portion. The peripheral surface comprises a first side portion and a second side portion which are interconnected by the end surface. The first side portion and the second side portion cooperate to define lateral extremities and width of the signal output portion. The width of the signal output portion is measured in a transverse direction which is orthogonal to the longitudinal axis and orthogonal to the first surface and/or the second surface. Where the signal output portion has a non-uniform width along its length, the width of the signal output portion is taken as the average width along the length.

The first surface has a facing orientation which is same as the facing orientation of the signal collection surface, that is, body-facing orientation. The second surface has a facing orientation which is same as the facing orientation of the second surface of the signal collection surface, that is, a non-body facing orientation. In example embodiments, the first surface and the second surface of the signal output portion are parallel (parallel herein includes substantially parallel) to each other and parallel to the longitudinal axis, and the end surface is orthogonal (orthogonal herein includes substantially orthogonal) to the longitudinal axis. In example embodiments such as the present, the end surface extends in a transverse direction which is orthogonal to the longitudinal axis and orthogonal to the first surface (or second surface). The end surface is on a signal output end of the signal output portion, and the signal output end is on a distal longitudinal end of the electrode.

In example embodiments such as the present, the signal output portion comprises a signal output pad which is singly formed as a conductive elastomeric pad. The first surface and the second surface of the signal output portion cooperate to define the thickness of the signal output pad. The thickness of the signal output portion is defined by the orthogonal separation between the first surface and second surface of the signal output pad. The thickness of the signal output pad is measured in a thickness direction which is a direction of orthogonal separation. The direction of orthogonal separation is orthogonal to the longitudinal axis and orthogonal to the first surface and/or the second surface. Where the signal collection portion has a non-uniform thickness along its length, the average thickness along its length is taken as the thickness.

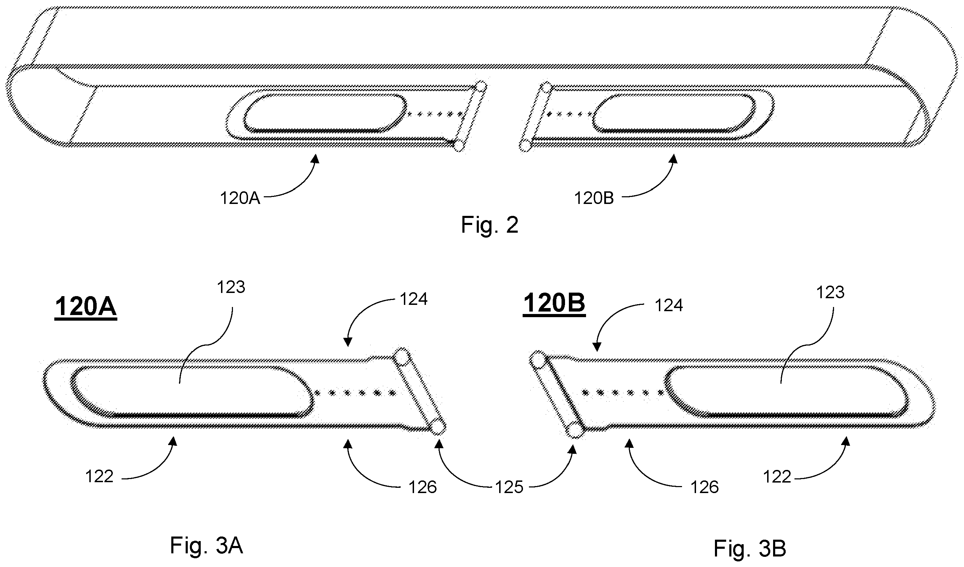

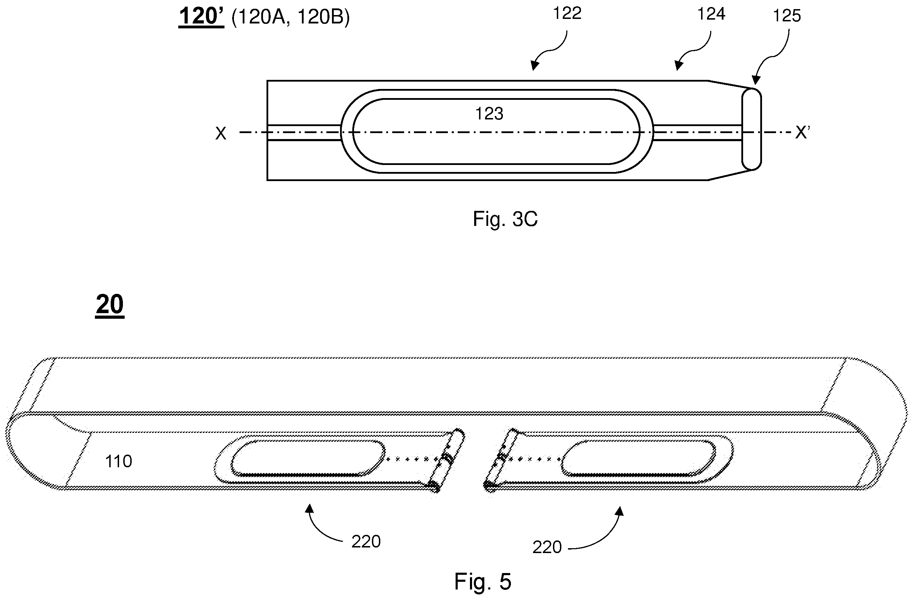

The signal output pad has a width. The width of the signal output pad is measured in a width direction. The width direction is orthogonal to the longitudinal axis and orthogonal to the thickness direction. The width of the signal output pad may be comparable to, smaller, or larger than the width of the signal collection pad. In example embodiments, the signal output pad has a uniform width throughout or along its length. In some embodiments, the signal output pad has a tapered end and tapers to narrow on extending towards the signal output end, as shown in Figure 3C.

The electrode comprises a signal output terminal. The signal output terminal is forward of the signal output pad and comprises a signal output surface which is configured to output signals collected by the signal collection portion.

The signal output surface extends between the first surface and the second surface of the signal collection pad and projects forward of the signal collection pad. The forward direction herein is a direction parallel to the longitudinal direction and from the signal collection portion towards the signal output portion. The forward direction is directly opposite to a rearward direction which is a direction parallel to the longitudinal direction, but from signal output portion to the signal collection portion.

The signal output surface extends transversely between the first side portion and the side portion of the peripheral surface. In example embodiments, the signal output terminal, or more particularly the signal output surface, extends transversely along an output terminal axis and intercepts the first side portion and the side portion of the peripheral surface. The output terminal axis is a transverse axis which is orthogonal to the longitudinal axis and orthogonal to the thickness direction of the signal output pad.

The signal output terminal is configured as a mechanical retainer so that the electrode can be retained by the electronic device, for example, without a fastener such as a mechanical fastener.

In example embodiments, the signal output surface projects to flare on extending forwardly and away from the signal output pad. The signal output surface may project at an acute angle with respect to the longitudinal axis on extending forwardly away from the longitudinal end of the signal output pad. In example embodiments, the signal output surface may flare to expand along a convex track or linearly on projecting forwardly and away from the longitudinal end of the signal output pad.

In example embodiments, the signal output surface comprises a first portion which begins from the first surface, a second portion which begins from the second surface, and a third portion which interconnects the first portion and the second portion. The third portion defines a forward-most surface of the electrode and also the forward-most surface of the signal output terminal. In example embodiments, the forward-most surface is a convex surface, for example, with respect to the signal output end of the signal output portion. The convexly curved forward-most surface extends to surround the signal output end, which is the forward longitudinal end of the signal output pad. In example embodiments, the signal output surface is a prism-defining surface (or prism surface in short) of a prism having a prism axis orthogonal to the longitudinal axis and orthogonal to the thickness direction. The signal output surface may continue along its entire width or may be interrupted at locations between its lateral ends.

The signal output surface of the signal output is an exterior surface which is exposed for making electrical connection with an electrical contact terminal, for example, by friction contact or compressive contact. An exterior surface herein means an outward-facing surface. The signal output surface has an outward-facing orientation and faces away from the signal output end and away from the signal input portion.

The signal output terminal and the signal output portion, or more particularly the signal output terminal and the signal output end of the signal output portion, cooperate to form a fastening portion. The fastening portion is configured to enter into detachable mated engagement with a counterpart fastening portion.

In example embodiments such as the present, the signal output terminal and the signal output portion, or more particularly the signal output terminal and the signal output end of the signal output portion, cooperate to form an anchor for anchoring on a retainer, and electrical connection is to be made between the retainer and the electrode when the anchor enters into mechanical engagement with the retainer.

To facilitate mechanical anchoring with electrical connection, the anchor may have a C-shaped, U-shaped, G-shaped, T-shaped, L-shaped, O-shaped, P-shaped, hook-shaped or cross-section of other shapes suitable to facilitate retention by insertion and removal by slide. The cross-section is taken along the longitudinal axis of the electrode as a whole.

In examples where the anchor has a T-shaped cross-section, the signal output terminal has a first limb and a second limb and the signal output pad forms a third limb which is a middle limb. When the T-shaped anchor is retained by a counterpart retainer, the first and second limbs, which are disposed on two sides of the middle limb, are retained by the retainer. The retainer and the signal output terminal may be configured as a pair of snap fasteners which can come into mated mechanical engagement when the retainer and the signal output terminal are bought into a relative mating position.

In examples where the anchor has a C-shaped cross-section, an O-shaped cross-section or a P-shaped cross-section, the entire cross-section is retained inside the retainer and makes electrical contact with the retainer.

In examples where the anchor has an L-shaped cross-section, the short limb is retained inside the retainer and makes electrical contact with the retainer.

To facilitate mechanical and electrical contact for signal output, the signal output terminal comprises a retention portion which is to cooperate with the retainer to form a pair of retention devices. In general, the retention portion comprises a portion which projects in the thickness direction and has a thickness which is substantially larger than the thickness of the signal output pad or the portion of the signal output pad at or near the signal output end. A substantially larger thickness herein means a difference in thickness of more than 20%, 25%, 30%, 35%, 40%, or a range or ranges selected from any combination of the aforesaid values.

In general, the signal output terminal is configured as an enlarged head portion at the signal output end of the electrode, and the enlarged head portion has a thickness larger than the thickness of the signal output portion to form a retainer. The enlarge head portion defines the longitudinal end, or more specifically the shape and dimensions of the of the longitudinal end electrode, and serves the dual purpose of a signal output surface and a retention device. The enlarged head portion may comprise a plurality of discrete head portions which are distributed in the transverse direction or may extend continuously along its length. In example embodiments, the enlarged head portion or each of the discrete head portions has a substantially uniform cross-section. The head portion or the discrete head portion may be a solid portion for enhanced robustness and retention strength. In example embodiments, the enlarged head portion is a solid prismatic conductive portion having a prism axis orthogonal to the longitudinal axis and orthogonal to the thickness direction.

The signal output terminal of the is attached to the signal output end, for example, by mechanical fastening, by soldering or by welding.

In examples not covered by the claimed invention, the signal collection portion, the bridging portion and the signal output portion are integrally moulded.

In some examples not covered by the claimed invention, the signal collection portion, the bridging portion, the signal output portion and the signal output terminal are integrally moulded.

In examples where the signal collection portion, the bridging portion and the signal output portion are integrally moulded as a single piece from a conductive material such as a conductive elastomer, the electrode may be covered by an insulating sleeve except at the signal collection surface and except at the signal output end.

In examples where the signal collection portion, the bridging portion, the signal output portion, and the signal output terminal are integrally moulded as a single piece from a conductive material such as a conductive elastomer, the electrode may be covered by an insulating sleeve except at the signal collection surface and except at the signal output terminal. The insulating sleeve may be made of a non-conductive elastomer and may be over-moulded onto the electrode. An electrode having an over-moulded elastomeric insulating sleeve has been water-resistant properties and may be used to collect signals in or under water, for example, during swimming.

The electrode may have a resistance of below 800ohm, 700ohm, 600ohm, 500ohm, 400ohm to facilitate in-water or under-water signal collection, especially for operation in sea water or chlorinated water. The resistance of the electrode is measured between the signal output terminal and the signal collection surface.

The example electronic signal collection device comprises a main housing, a plurality of signal input terminals mounted on the main housing, and electronic circuitry mounted inside the main housing. The electronic circuitry may have different circuit configurations according to requirements. For example, the electronic signal collection device may be configured as a signal transmitter for transmitting physiological signals collected by the electrodes to an external device for signal processing, as a performance monitor having a processor to processor physiological signals collected by the electrodes and for outputting processed data, and/or as a performance controller to transmit signals to the body surface. In general, the electronic circuitry would comprise solid-state circuits including processors such as a microcontroller or a microprocessor, data storage devices such as memories including volatile and non-volatile memories, telecommunication frontend devices including an RF receiver and/or an RF transmitter. The electronic circuitry may comprise a small battery, such as a CR2032 battery to provide operation power.

In example embodiments such as the present, the electrode is configured to collect ECG signals as an example of physiological signals and the electronic circuitry is configured to process ECG signals collected by the electrodes to output physiological data such as heart rate and salient ECG characteristics such as data for constructing the RST wave portions.

The example apparatus is configured as a chest strap for collecting ECG signals from the left-rib cage and right-rib cage of a wearer. When configured and used as a chest strap, or ECG chest strap, the electronic signal collection device is to be positioned between the two rib cages, with the signal collection surface of the first electrode in abutment contact with the left rib cage and the signal collection surface of the second electrode in abutment contact with the right rib cage. Alternatively, the first electrode may be in abutment with the right rib cage and the second electrode may be in abutment with the left rib cage without loss of generality.

The main housing of the example electronic signal collection device comprises a plurality of receptacles for fitted reception, including snap-fit reception, of a corresponding plurality of signal output terminals of the electrodes. When the signal output terminal is in fitted reception within the receptacle, the signal output surface of the electrode is in compressive contact or frictional contact with the signal input surface of the signal input terminal of the signal collection device. Each receptacle has an internal wall which defines an internal compartment. The internal compartment is shaped and dimensioned for closely-fitted reception of a signal output terminal of an electrode. The signal input terminal is mounted on the main housing and has a signal input surface which is exposed inside the receptacle for making physical contact and electrical contact with the signal output surface of the electrode. The internal wall may be a conductive wall which is to function as a signal input surface of the signal input terminal. In some embodiments, the signal input terminals may comprise a resiliently biased contact pin or a plurality of resiliently biased contact pins which are to retract under resilient bias when the signal output terminal encounters the contact pin on entry into the receptacle. The main housing may include latching means to latch the signal output terminal when the signal output terminal has reached a predetermined latching position with respect to the receptacle. The signal output terminal would need to overcome a larger force than the force to insert into the receptacle when a user is to remove the signal output terminal from the receptacle to separate the electrode from the main housing. The receptacle has a receptacle axis. The receptacle axis and the output terminal axis are coaxially aligned when the signal output terminal is snap retained by the receptacle.

In example embodiments such as the present, the receptacle may comprise an entry aperture on a receptacle end to permit slide entry of the signal output terminal, or more specifically to permit slide entry of the enlarged head portion of the signal output terminal into the internal compartment of the receptacle. To permit movement of the signal output terminal into a fully retained position, the receptacle comprises a slit which begins from the entry aperture and extends in a direction parallel to the receptacle axis. In some embodiments, the receptacle has entry apertures on the receptacle ends.

In example embodiments such as the present, the main housing comprises a first receptacle and a first signal input terminal on a first lateral side and a second receptacle and a second signal input terminal on a second lateral side. A first signal input terminal is formed on the first receptacle and a second signal input terminal is formed on the second receptacle. When the electrodes are in place and in detachable mechanical engagement with the electronic signal collection device, the first electrode, the main housing and the second electrode are in series and distributed along the longitudinal axis. The main housing may be made of hard plastics and formed as a watertight container to house the electronic circuitry.

The plurality of physiological signal collection electrodes comprises a first physiological signal collection electrode **120A** (or first electrode in short) and a second physiological signal collection electrode **120B** (or second electrode in short). The first electrode is physically and electrically connected to the first signal input terminal and the second electrode is physically and electrically connected to the second signal input terminal.

When the electrodes are in mechanical and electrical connection with the signal collection device, the electrodes are disposed on two sides of the main housing in a symmetrical manner, so that the signal output potions of the electrodes are proximal to the signal collection device and the signal collection portion are distal from the signal collection device. When used in this chest strap configuration, the longitudinal extent of the signal collection assembly, comprising the signal collection device and the signal collection electrodes, is defined by the longitudinal separation between the longitudinal ends of the signal collection portions which are distal from their corresponding or respective signal output ends.

In example embodiments, the carrier is a flexible strap, for example, a flexible fabric strap. In some embodiments, the strap has longitudinal ends and the electrodes are mounted on the longitudinal ends of the fabric strap or other portions of the strap without loss of generality. In some embodiments, the strap is formed as a loop and the electrodes may be mounted on the loop. Where the electrodes are mounted on the longitudinal ends of the flexible strap, a closed loop is formed when the electrodes are fastened to the electronic device.

The fabric strap may comprise both elastic portions and non-elastic portions. Alternatively, the fabric strap may comprise of all elastic portions. The strap may be made of woven and/or non-woven materials.

The first and second electrodes are mounted on the carrier to form a strap assembly. In example embodiments, the signal output terminals form the longitudinal ends of the strap assembly. The apparatus is assembled when the signal output terminals of the electrodes are attached to the electronic device. The apparatus is disassembled when the signal output terminals of the electrodes are detached from the electronic device. To assemble the apparatus, a user is to insert the signal input terminals into the receptacles on the electronic device and to slide the signal input terminal in the direction of insertion until the signal input terminals are retained by the receptacle. When the signal input terminals are mechanically retained by the receptacles, for example, by friction retention, latched retention, snap retention, or other forms of retention, electronic connection to facilitate signal communication between the electrode and the electronic device is established. To disassemble the apparatus, a user is to slide the signal input terminals in a direction opposite to the insertion direction until the signal input terminals are detached from the receptacle. When the signal input terminals are detached from the receptacles, electronic signal communication between the electrode and the electronic device is cut.

A signal output terminal made of a conductive elastomer and configured also as a retention portion is advantageous since it provides enhanced frictional contact with the receptacle and signal input surface of the electronic device and hence more secured retention while permitting removal without the assistance of a tool.

A signal output terminal which is integrally formed as single piece with the signal output pad or integrally formed as a single piece with the signal collection portion is advantageous for enhanced reliability as well as promoting signal transmission continuity and mitigate signal loss or distortion.

In example embodiments, the signal output terminal is fastened to the signal output pad.

An example signal output terminal **260** comprises a conductive main body **262** and a channel **264** extending through the main body, as depicted in Figures 6C1, 6C2 and 6C3.

The main body comprises an inner peripheral wall which defines a channel and an outer peripheral wall which surrounds the channel and which defines a signal output surface. The channel extends along a channel axis Y-Y' and has a length equal or comparable to the output end of the signal output pad. The channel axis Y-Y' is orthogonal to the longitudinal axis X-X' and orthogonal to the thickness direction of the electrode. The channel has a clearance in the thickness direction of the electrode and the channel clearance in the thickness direction is smaller, for example, slightly smaller than the thickness of the signal output end of the signal output pad. In example embodiments, the channel axis Y-Y' is a centre axis of the main body and the main body is symmetrical about the channel axis. The channel is configured for closely-fitted reception of the signal output end of the signal output pad. In example embodiments, the main body is fastened to the signal output end of the signal output pad by a fastener or a plurality of fasteners. The fastener may be a threaded screw or a rivet.

The main body has a generally C-shaped or U-shaped cross-section, with the entry aperture at the opening portion of the C-shaped or U-shaped cross-section. The entry aperture and/or the interior slot is/are shaped and/or dimensioned for fitted (or closely fitted) reception of the output end of the signal transmission pad. To facilitate fitted (or optionally closely fitted) reception of the output end of the signal transmission pad, the entry aperture and/or the interior slot has a transversal clearance which is comparable or slightly smaller than the thickness of the output end of the signal transmission pad. The transversal clearance of the entry aperture and/or the interior slot is measured in a direction (the transverse direction) which is orthogonal to the longitudinal direction (or overall longitudinal direction) of the longitudinal direction defined by the longitudinal axis X-X'. The main body of the signal output terminal **126** is laterally symmetrical about a center axis Z-Z', and the center axis Z-Z' is orthogonal to both the X-X' and Y-Y' axes.

According to the invention, the main body of the signal output terminal **126** is a hollow metal bar, for example, a copper bar, an aluminum bar, a stainless-steel bar or other a bar of other metals or alloys. In some examples not covered by the claimed invention, the main body is made of a moldable material such as a conductive polymeric material, for example, carbonized rubber. For example, the main body may be formed of the same materials as the materials forming the signal collection pad and/or signal transmission pad. In some examples, the signal collection pad and/or signal transmission pad and/or the main body of the output terminal are integrally formed as a single flexible piece.

In some embodiments, the main body may be integrally made of non-metal, for example, conductive elastomer.

The main body of the example signal output terminal is shaped and dimension for closely fitted reception of the output end of the signal transmission pad. To facilitate the closely fitted reception, the main body of the example signal output terminal and the output end of the signal transmission pad are a well-matched snap-fit pair, and the main body of the example signal output terminal is shaped and dimension to function as a clamp or clamping member to clamp on the output end of the signal transmission pad. As the main body of the example signal output terminal has a C-shaped cross section, the main body and/or the signal output terminal is also a C-clamp shaped electrical connector. When the C-clamp connector is attached to the signal transmission pad, the output end of the signal transmission pad is guarded, covered or protected to enhance durability.

As the main body of the example signal output terminal and the output end of the signal transmission pad are a well-matched snap-fit pair, the internal boundary wall is contiguous or abutment contact with the output end of the signal transmission pad. Since the internal boundary wall of the signal output terminal is in contiguous or abutment contact with the output end of the signal transmission pad, the internal boundary wall of the signal output terminal, which defines the entry aperture and the interior compartment, defines a distributed signal input portion of the signal output terminal. The internal boundary wall of the signal output terminal includes an upper boundary wall, a lower boundary wall and a peripheral wall interconnecting the upper and lower boundary walls. When the signal output terminal is mounted on the signal transmission pad, which effectively means when the signal output terminal is mounted on the output end of the signal transmission pad, the upper and lower surfaces on the output end of the signal transmission pad are in contiguous or abutting physical and electrical contact with the upper and lower boundary walls of the signal output terminal. When the signal output terminal is mounted on the signal transmission pad, the output end of the signal transmission pad is in compressive contact with the signal output terminal. More specifically, the output end of the signal transmission pad is compressively sandwiched between the upper and lower boundary walls. Each one of the upper and lower boundary walls is an elongate conductive wall and the upper and lower boundary walls cooperate to define an elongate, distributed signal input of the signal output terminal.

When the physiological signal apparatus is duly worn on the living body, with the signal collection electrodes in abutment contact with different body portions, ECG signals will be collected by the ECG signal collection electrodes and transferred to the electronic apparatus via the signal output terminals and the signal input terminals.

In some embodiments, the signal input terminal inside the signal input receptacle may be a distributed connector. For example, the signal input terminal may include a tubular metallic portion which is to resiliently receive and engage with the signal output terminal to facilitate signal transfer without loss of generality.

An example of another physiological signal monitoring apparatus **20** comprises a strap **110** and a pair of physiological signal collection electrodes 220 mounted on the strap, as depicted in Figures 5.

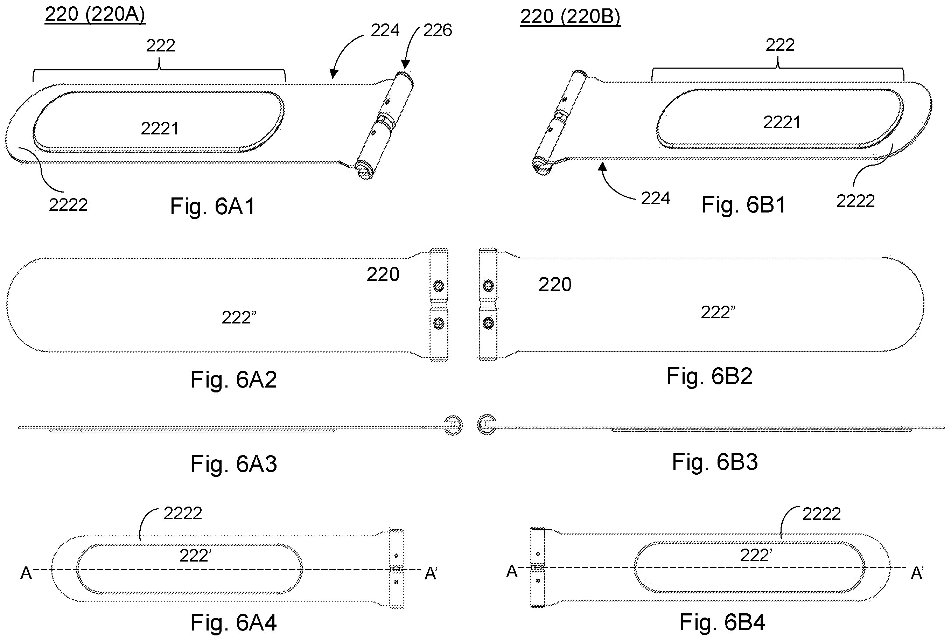

The pair of example physiological signal collection electrodes comprises an example first signal collection electrode **220A** and an example second signal collection electrode **220B,** as depicted in Figures 6A1 to 6A4 and 6B1 to 6B4. Each example physiological signal collection electrode **220** comprises a signal collection portion **222,** a signal output terminal **226,** and a signal transmission bridge **224** interconnecting the signal collection portion and the signal output terminal. The signal collection portion **222,** the signal transmission bridge **224** and the signal output terminal **226** are distributed sequentially along a central axis A-A' of the signal collection electrode, and the central axis is also a longitudinal axis which defines a longitudinal direction of the electrode.

The signal collection portion **222** is a signal collection pad comprising a signal collection surface **222'** for making abutment contact with the body of a wearer to facilitate collection of physiological signals from the body surface, the signal collection surface is an exposed surface which is parallel to and faces away from the local strap surface on which the signal collection portion **222** is mounted. The signal collection portion **222** has a back surface **222"** which is parallel to and in abutment contact with the local strap surface. The signal collection surface **222'** has a signal collection area which is large enough to collect physiological signals having a sufficiently large signal magnitude suitable for output and for processing by another electronic device **40.** The signal collection surface **222'** is generally parallel to the longitudinal axis of the signal collection electrode **220** and the longitudinal axis of the signal output terminal, which is orthogonal to the longitudinal axis of the signal collection electrode **220,** the longitudinal axis of the signal collection electrode **220** and the longitudinal axis of the signal output terminal cooperating to define a signal collection plane.

In embodiments such as the present, the signal collection portion comprises an elevated signal collection surface portion which is elevated from a base signal collection surface portion, which surrounds the elevated signal collection surface portion so that the elevated signal collection surface portion defines a signal collection island **2221** surrounded by the base signal collection surface portion **2222.**

In embodiments such as the present, the signal collection portion **222,** comprising the signal collection island **2221** and the surrounding base portion **2222,** are integrally formed from a flexible and conductive moldable soft material such as carbonized rubber or carbonized plastics. The signal collection portion **222** may be made of a flexible and conductive fabric, including woven and non-woven fabrics. The signal collection portion has a generally uniform thickness along its width, the width being defined in a direction orthogonal to the longitudinal direction of the electrode. Where the signal collection portion has a signal collection island **2221,** the signal collection island **2221** is a signal collection pad having a generally uniform thickness and a generally uniform width, and the surrounding base portion **2222** has the same or another generally uniform thickness and width.

The signal transmission bridge **224** is to facilitate conduction of electrical signals between the signal collection portion **222** and the signal output terminal **226.** In example applications, physiological signals, in the form of low-frequency electrical pulses collected by the signal collection portion, are to be transmitted forwardly to the signal transmission bridge **224** and then forwardly to the signal output terminal **226** for ultimate output. The electrical signals are to travel in the longitudinal direction of the electrode **220,** the longitudinal direction defining an electrical conduction path of the shortest distance between the signal collection portion **222** and the signal output terminal **226.** The example signal transmission bridge **224** is made of a flexible and conductive material and comprises a flexible bridging portion which extends between the signal collection surface and the signal output terminal. The bridging portion comprises a body-facing surface which is parallel to the signal collection surface and an outward-facing surface which faces away from the wearer during use. The bridging portion continues electrically and physically from the forward longitudinal end of the signal collection portion and the signal output terminal is connected to the forward longitudinal end of the signal transmission bridge **224.** In embodiments such as the present, the signal collection portion **222** and the signal transmission bridge **224** are integrally formed, for example, molded, from the same material. The signal collection portion **222** and the signal transmission bridge **224** may have the same width, although in some embodiments the signal transmission bridge **224** has a smaller width than the signal collection portion **222.** The signal transmission bridge **224** may have a width which is 65-85% of the width of the signal collection portion **222.**

Each of the signal collection portion **222** and the signal transmission bridge **224** is elongate and has a low-profile form factor. A low-profile form factor herein means having a width which is substantially larger than its thickness. The signal collection pad (or pad in short) and the bridge portion resembles the form factor of a strip of an elongate tab. The example pad and bridge portion have an example width-to-thickness ratio of ≥ 3: 1, 4: 1 *or* 5: 1. In example embodiments, the signal collection portion and the signal transmission bridge are integrally formed, for example, from a flexible and resilient conductive material such as carbonized rubber so that the signal collection electrode **220** is in the form of an elongate strip or tab. In example embodiments such as the present, the pad and the bridge portion have substantially same or uniform thickness along the longitudinal axis.

The strap **20** has a first longitudinal end **22A,** a second longitudinal end **22B** and an intermediate portion **22** interconnecting the first and second longitudinal ends **22A, 22B,** and the intermediate portion **22** defines an effective length of the strap along its center longitudinal axis. The first signal collection electrode **220A** is mounted on the intermediate portion **22** of the strap **20,** with its signal output terminal **226** proximal and terminating the first longitudinal end **22A.** The signal output terminal **226** extends orthogonally to the longitudinal axis of the signal collection electrode **220A** and parallel to the signal collection surface to encapsulate the signal output end portion of the first signal collection electrode **220A.** The signal output terminal **226** encapsulates the entire width of the signal output end portion of the first signal collection electrode **220A** as depicted in Figures 6A1 to 6A4, or a substantial width portion thereof. The signal collection portion **222** of the first signal collection electrode **220A** is mounted on the intermediate portion **22** of the strap and extends longitudinally away from the first longitudinal end **22A** and longitudinally towards the second longitudinal end **22B** in a direction opposite to the direction extension of the second signal collection electrode **220B.** The example signal output terminal **226** has a generally C-shaped cross-section along its length to form a C-shaped clamp and the signal output end portion of the signal collection electrode **220A, 220B** is retained inside the internal compartment of the C-shaped clamp and is compressively engaged so that good electrical contact is made between the signal output end portion and the signal output terminal **226** to facilitate effective and efficient physiological signal transfer.

Similarly, the second signal collection electrode **220B** is mounted on the intermediate portion **22** of the strap **20,** with its signal output terminal **226** proximal and terminating the second longitudinal end **22B,** and with the signal output terminal **226** extending orthogonally to the longitudinal axis of the signal collection electrode **220B** and parallel to the signal collection surface to encapsulate the signal output end portion of the second signal collection electrode **220B.** The signal output terminal **226** encapsulates the entire width of the signal output end portion of the second signal collection electrode **220B** as depicted in Figures 6B1 to 6B4, or a substantial width portion thereof. The signal collection portion **222** of the second signal collection electrode **220B** is mounted on the intermediate portion **22** of the strap and extends longitudinally away from the second longitudinal end **22B** and longitudinally towards the first longitudinal end **22A** in a direction opposite to the direction extension of the first signal collection electrode **220A.**

In embodiments such as the present, each one of the first and second longitudinal ends **22A, 22B** is a free end so that the strap **20** is convertible between an open-ended belt and a closed loop when in cooperation with a strap-ends connector (or strap connector in short). The strap **20** may be made of fabrics, for example, a breathable, non-conductive and resiliently stretchable fabric, including woven and non-woven fabrics. The strap **20,** or more specifically the immediate portion **22** of the strap, is intended to function as an insulated carrier or an insulated substrate of the signal collection electrodes **220A, 220B** and the associated electronic device. The intermediate portion **22** of the strap **20** provides partial physical shielding to the back surface of the signal collection electrode, and more specifically to the signal collection pads and the bridge portions so that the pads and bridge portions of the signal collection electrodes **220A, 220B** are visually hidden when viewed towards the user with the electronic device in sight. In addition, the intermediate portion **22** of the strap is to mechanically shield the pads and the bridge portions from at least a substantial portion of the longitudinally elongating tension to promote durability and reliability, since the end of the electrode distal from the signal output terminal is floatingly carried on the intermediate portion and is not fastened to a longitudinal end of the strap. The term floatingly herein means portion of the intermediate portion of the strap between the distal ends of the electrodes is more elongate-able then the portions which are in abutment with the pads and the bridging portions.

The strap **20** and the pair of signal collection electrodes **220A, 220B** mounted thereon collectively form a signal collection strap which is also a signal collection apparatus **230.** In embodiments such as the present, the signal collection apparatus **230** is a passive apparatus having passive components for passive collection of physiological signals. The signal collection apparatus is to cooperate with an electronic device to facilitate signal processing, data storage and/or for on-ward transmission of signals or data. In example embodiments such as the present, the electronic device is configured as a strap-ends connector which converts the open-ended strap apparatus into a closed-loop signal collection and processing apparatus.

The electronic device **40** which is to function as a strap-ends connector is also a physiological signal hub (signal hub in short). Physiological signals collected by the first and second electrodes **220A, 220B** are to flow into the electronic device **40** and the electronic circuitry thereof is to process the collected signals according to the design of the electronic circuitry. The electronic circuitry may comprise signal processing circuitry, data storage circuitry and/or data transmission circuitry for onward transmission of raw or processed data.

In example embodiments such as the present, the electronic device **40** and the electrodes **220A, 220B** are in physical and electrical connection so that collected physiological signals are to flow into the electronic circuitry and processed by circuitry therein.

The electronic device **40** comprises a rigid main housing **42,** a first hub terminal **44A** on a first side of the main housing forming a first retention terminal and a second hub terminal **44B** on a second side of the main housing forming a second retention terminal, the second hub terminal being on a diametrically opposite side of the first hub terminal, as depicted in 7A1-7A4. The main housing **42** defines a hollow internal compartment inside which the electronic circuitry is housed. The first hub terminal **44A** comprises a first retention device for retention of the first signal output terminal and a first signal input terminal of the signal hub. The second hub terminal **44B** comprises a second retention device for retention of the second signal output terminal and a second signal input terminal of the signal hub. During normal use when the assembly in a physiological signal collection mode and when physiological signals are to be collected from a user or wearer, the signal collection apparatus is in an anchored mode and is anchored on the electronic device **40** as a signal hub, with the first signal output terminal anchoring on the first retention terminal and with the second signal output terminal anchoring on the second retention terminal. When in the anchored mode, the first signal output terminal **226** of the first signal collection electrode **220A** is mechanically retained by the first retention device of the signal hub and is prevented from separation in the longitudinal direction even when the first signal output terminal **226** is subject to a longitudinal separation force, and the second signal output terminal **226** of the second signal collection electrode **220B** is mechanically retained by the second retention device of the signal hub and is prevented from separation in the longitudinal direction even when the second signal output terminal **226** is subject to a longitudinal separation force opposite to the longitudinal separation force on the first signal collection electrode **220A.**

In embodiments such as the present, the output terminal **226** is for snap-fit latched engagement with the first retention device of the hub terminals **44A, 44B.** To facilitate snap-fit latched engagement, the output terminal **226** and the hub terminals **44A, 44B** respectively comprise complementary snap-fit latching components and/or have complementary shapes and dimensions to facilitate closely-fitted reception and to enhance snap-fit latching.

In embodiments such as the present, the output terminal **226** and its corresponding retention terminal on the hub terminals **44A, 44B** are relatively movable along a traverse direction to the strap longitudinal axis so that the output terminal **226** can move into or out of mechanical engagement with a corresponding retention terminal.

When the signal collection apparatus **230** is in anchored engagement with the signal hub **40,** the signal collection assembly comprising the signal collection apparatus **230** and the signal hub **40** is in the configuration of a closed loop. Therefore, the first retention terminal also functions as a first anchoring terminal and the second retention terminal also functions as a second anchoring terminal. The intermediate portion **22** of the strap **20** is in the form of an elastic band and is flexible, resilient and stretchable or elongate-able in the longitudinal direction. When in normal use, the strap assembly comprising the signal collection apparatus and the signal hub is worn as a closed loop on the body of the user, with the intermediate portion of the strap subject to an elongating tension in the longitudinal direction and with the signal collection surfaces of the signal collection electrodes in abutment contact with spaced apart naked body portions of the user. When in the normal use configuration, the signal collection surfaces of the signal collection electrodes **220A, 220B** will be in physical compression to establish electrical contact with the respective exposed body surfaces of the spaced apart body portions of the user. The length of the intermediate portion of the strap is adjusted according to the size of the user, or more specifically the size of the body portion of the user, in order to produce an appropriate level of radial compressive tension for effective electrical signal collection from the exposed body surface of the user. The strap may be woven from elastic yarns to provide elasticity and breathability. To enhance comfort and breathability, the signal collection portion and/or the signal transmission portion may be perforated.

The signal output terminal **226** of the signal collection electrode **220** comprises a main body and a receptacle. The main body is elongate and extends along a longitudinal direction between a first longitudinal end and a second longitudinal end, the longitudinal direction being defined by a longitudinal axis which is also a center axis of the main body, and the longitudinal direction of the main body is orthogonal or substantially orthogonal to the longitudinal direction of the signal collection electrode **220.** The main body has an outer peripheral wall defining an outer peripheral surface and interconnecting the first and second longitudinal ends.

The receptacle comprises an open channel for receiving a signal output end portion of the signal collection electrode **220.** The open channel is elongate, extends between the first and second longitudinal ends, and has a channel axis which is a center axis of the channel. In example embodiments such as the present, the channel axis is parallel to the longitudinal axis of the main body. The open channel has an entry aperture formed on the first longitudinal end and an electrode bridge passage aperture formed on the outer peripheral surface. The electrode bridge passage aperture is defined by a bridge passage window which is formed on the outer peripheral surface. The bridge passage window has a width which is wider, for example slightly wider, than the width of the signal output end portion of the signal collection electrode and a depth which is larger, for example slightly larger, than the thickness of the signal output end portion of the signal collection electrode. In general, the bridging portion passage window defines a transversal aperture which is a window aperture having a clearance or clearance dimensions sufficient to permit slide entry of the portion of the bridging portion which is immediately proximal the signal output end portion into the bridge passage window by moving or sliding in a lateral or transversal direction (which is parallel to the longitudinal direction of the main body) while preventing through-passage of the signal output terminal across the window by moving in the longitudinal direction of the signal collection electrode (which is orthogonal to the longitudinal direction of the main body).

In embodiments such as the present, the open channel extends between a first entry aperture which is formed on the first longitudinal end and a second entry aperture which is formed on the second longitudinal end. In addition, the aperture defined by the bridging portion passage window is a through aperture extending between the first and second longitudinal ends. When the aperture is a through aperture on the outer peripheral surface of the main body, the portion of the bridging portion which is immediately proximal the signal output end portion can slide into the bridge passage window into the anchoring terminal in either end, that is opposite, transversal directions.

The main body of the signal output terminal **226** comprises an upper body portion, a lower body portion and a forward body portion interconnecting the upper and lower body portions. Each of the body portions is elongate and extends in the longitudinal direction of the main body and the body portions cooperate to define the open channel and the window aperture. The upper body portion has an upward-facing upper surface which is an outer surface in abutment contact with the anchoring terminal and a downward-facing lower surface which is an interior surface, the downward-facing surface defining a ceiling portion of the open channel. The lower body portion has an upward-facing upper surface which is an interior surface defining a floor portion of the open channel and a downward-facing lower surface which is an outer surface in abutment contact with the anchoring terminal. The forward body portion forms the forward (or forward-most) end of the signal collection electrode and comprises a forward-facing surface which is in abutment contact with the anchoring terminal and a rearward-facing surface which is opposite facing and proximal the longitudinal end of the bridging portion. The forward body portion orthogonally intercepts the center longitudinal axis of the signal collection electrode. The upper body portion, the lower body portion and the forward body portion cooperate to define an internal compartment for receiving a signal output end portion of the signal collection electrode **220.**

In embodiments such as the present, the upward-facing surface of the upper body portion is an upper surface of the signal output terminal and is a convex surface with respect to the center axis of the main body, the downward-facing surface of the lower body portion is a lower surface of the signal output terminal and is a convex surface with respect to the center axis of the main body, and the forward-facing surface of the forward body portion is the forward-most surface of the signal collection electrode and is a convex surface with respect to the center axis of the main body. Optionally, the curved surfaces of the outer surfaces of the body portions of the main body share a common radius of curvature with respect to the center longitudinal axis of the main body, so that the main body is a cylindrical body, which means a generally and not necessarily absolutely cylindrical body herein. In some embodiments, the main body has an oval, square or polygonal cross section.

The upper body portion of the signal output terminal projects upwardly to protrude above the upper major surface of the signal transmission bridge while the lower body portion of the signal output terminal projects downwardly to protrude below the lower major surface of the signal transmission bridge, the upper and lower major surface cooperating to define thickness of the bridging portion of the signal transmission bridge.

A forward longitudinal end portion of the signal transmission bridge or bridging portion is received inside the open channel for forward transmission of electrical signals collected by the signal collection electrode to the electronic device connected to the pair of signal collection electrodes. The forward longitudinal end of the portion of the signal transmission bridge is retained inside the open channel, for example, by compressive clamping force exerted by cooperation of the upper and lower body portions and/or by means of fastening means.

In example, embodiments, the fastener means comprises a screw or a plurality of screws which penetrates through the upper or lower body portion of the signal output terminal in a direction orthogonal to both the main body center axis and the electrode center axis to engage with the forward longitudinal end portion of the signal transmission bridge, which is a signal output end portion of the signal collection electrode.

In some embodiments, the upper body portion and/or the lower body portion of the signal output terminal is/are in compressive engagement with the signal transmission bridge to facilitate efficient signal transfer between the signal collection electrode and the electronic device.

In some embodiments, the forward end of the signal collection electrode is mounted on the strap end as a substrate, in which case at least the upper major surface of the signal transmission bridge, which faces the wearer during use, is in compressive engagement with the upper body portion of the signal output terminal.

In example embodiments, the length of the main body is comparable to or approximately equal to the width of the bridging portion or the width of the forward longitudinal end of the portion of the signal transmission bridge.

In example embodiments such as the present, the main body is made of a highly conductive metal such as copper, copper alloy, nickel and/or chromium alloy such as a nickel or chromium steel.

In example embodiments such as the present, the main housing **42** of the electronic device **40** comprises an upper housing portion and a lower housing portion which are made of hard plastics and are fastened together to form a water-tight housing to define an internal circuitry compartment for housing the electronic circuitry. The man housing **42** comprises a peripheral wall which surrounds the internal compartment, as depicted in Figures 7A1 to 7A4. The hub terminal **44A, 44B** of the electronic device **40** comprises a hub terminal housing which defines an output terminal receptacle **44A2, 44B2** for mechanical retention of and electrical connection with the signal output terminal **226** of the signal collection electrode. The hub terminal housing projects laterally outwards from a lateral side of the main housing **42** and the hub terminals **44A, 44B** are on opposite diametrical sides of the circuitry compartment, the opposite diametrical sides being on the longitudinal axis of the signal collection electrodes. The hub terminal is a lateral extension of the main housing **42** of the electronic device and defines an output terminal receptacle for receiving the signal output terminal **226** of a signal collection electrode **220A, 220B.**

The receptacle comprises a channel for receiving the signal output terminal **226,** a strap passage aperture **44A4, 44B4** to permit a portion of the strap **22** to pass through the receptacle, and an entry aperture to permit the signal output terminal **226** to enter into the receptacle, as depicted more clearly in Figures 8A1 to 8A3. The example channel of the hub terminal **44A, 44B** is an open channel, with entry aperture defining an entry end of the open channel, so that the signal output terminal **226** can slide into the receptacle along the longitudinal direction of the signal output terminal **226** and through entry at the entry aperture on a longitudinal end of the channel. The channel has a center axis which is orthogonal to the longitudinal axis of the signal collection electrode **220A, 220B.** The strap passage aperture is defined by a pair of aperture-defining walls each having an edge parallel to the channel center axis to define an elongate strap passage aperture. The dimensions of the strap passage aperture, in particular the height, are large enough to permit through-passage of the strap in the longitudinal direction but are not large enough to permit through-passage of the signal output terminal **226** in the longitudinal direction. The receptacle has an internal compartment having dimensions comparable to that of the signal output terminal **226** for closely fitted reception. The receptacle has internal walls which defines the open channel. Exposed electrical contacts for making electrical contact with the signal collection electrode **220A, 220B** are formed on and/or protruding radially inwards from internal walls.

When the signal output ends of the signal collection apparatus **230** are received inside the hub terminals **44A, 44B,** the longitudinal axis of the output signal terminal is aligned (that is coaxial) with the center axis of the output terminal receptacle **44A2, 44B2.** The signal output end portion of the signal collection apparatus **230,** comprising longitudinal end portions of the signal collection electrode **220A, 220B** and the strap **22,** is mechanically fastened to the signal output terminal **226** by fastening means. The fastening means penetrates through the signal output end portion of the signal collection apparatus **230** and anchors on the upper and lower body portions of the signal output terminal **226,** as depicted in Figures 8A1 and 8A2. The example fastener means comprises a pair of screws, and each screw has a shank which extends orthogonally through the signal output end portion of the signal collection apparatus **230.** The signal output end portion of the signal collection apparatus **230** occupies almost all the space inside the hub terminals **44A, 44B** to ensure good electrical contact with the exposed electrical contacts **446** inside the hub terminals **44A, 44B** and the exposed electrical contacts **446** are in electrical connection with the electronic circuitry of the electronic device **40.**

A localized indentation or dent is formed on the outer peripheral surface of the main body of the signal output terminal **226.** In the present example embodiment, the dent is formed on the middle of the main body and the exposed resilient contact **446** comprises a rounded contact peak which projects upwardly from a lower body portion of the output terminal receptacle **44A2, 44B2** to complementarily engage with the dent on the signal output terminal **226.**

Referring to Figure 8A3, exposed resilient contacts **446** are formed inside the receptacle and protrudes radially inwards to resilient engage with the signal output terminal **226** to facilitate good mechanical and electrical contact by friction engagement for efficient signal transfer. The contact **446** is formed from a metal strip which is bent into the shape having a wider base in abutment with the output terminal receptacle **44A2, 44B2** and a round contact peak projecting radially into the open channel and elevated from an inner base surface of the output terminal receptacle **44A2, 44B2.**

In embodiments such as the present, the signal collection electrodes are for collecting ECG signals and the signal collection surface has an example length of between 8cm-20cm and a width of between 1.5cm-2.5cm, with rounded corners at the longitudinal ends. The bridging portion has an example length of between 5cm-12cm and a width of between 1.0 -2.2 cm.

In embodiments such as the present, the apparatus and the electrodes are for collecting and processing low-frequency electrical pulses such as ECG pulses or muscle contraction pulses having a frequency range of between tens to hundreds or cycles. Of course, electronic circuitry for processing higher-frequency components of the physiological signals may be included without loss of generality.

In some embodiments, the signal collection portion **222,** the signal transmission bridge **224** and the signal output terminal **226** are integrally formed as a single piece, for example, from conductive rubber or soft metal or conductive fabrics.

In example embodiments, the signal collection surface is not elevated above the base region of the signal collection portion. For example, the signal collection surface may be flush or leveled with the base region of the signal collection portion. In some embodiments, the signal collection island or the non-elevated signal collection surface may be defined by an insulating sheet which delineates a signal collection island and a signal collection surface. In some embodiments, the signal output terminal of an electrode which has a non-elevated signal collection surface is integrally formed with the signal output portion. A signal output terminal which is integrally formed with the signal output pad promotes signal continuity and signal reliability, as well as enhanced robustness.

While the present disclosure has been explained with reference to example or embodiments, it should be appreciated that the embodiments are only described to illustrate the invention and are not meant for restricting the scope of invention. For example, while a swim monitor strap has been illustrated as an example, a strap of the present invention could be used for running or other activities without loss of generality.

While the example physiological signal apparatus **200** is a performance monitoring apparatus comprising a signal collection electrode **220,** the apparatus may be adapted to be used in a stimulation system. When adapted to be used in a stimulation system, signal collection electrode may be adapted to operate as stimulator for stimulating a tentative surface. When adapted to function in a stimulation system, the electronic device **40** may comprise an antenna for receiving instructions from an external source. The electric device generates signal, which is transferred to the stimulator. The stimulation system may be used in, for examples, electrical stimulation treatment for obstructive sleep apnoea, electrical muscle stimulation for physical therapy or psychological research on animals.

## Claims

1. A physiological signal collection electrode comprising a signal collection portion (122, 222), a signal output portion (124, 224) having a signal output end, and a bridging portion interconnecting the signal collection portion and the signal output portion;
wherein the signal collection portion (122, 222), the bridging portion and the signal output portion (124, 224) are distributed in series along a longitudinal axis and the signal output portion is on a longitudinal end of the longitudinal axis;
wherein the signal collection portion comprises a signal collection surface for collection of physiological signals from a living body by body contact,
wherein the signal output portion comprises a signal output pad having a forward longitudinal end and a signal output terminal (126, 226) at the forward longitudinal end of the signal output pad;
wherein the signal output pad has a first surface, a second surface, and a peripheral surface that includes an end surface which is a forward-end surface interconnecting the first surface and the second surface, wherein the signal output terminal is in physical and electrical contact with both the first surface and the second surface, and has a signal output surface which is configured to output signals collected by the signal collection portion and which is an exterior surface that is exposed for making electrical connection and extends about a traverse axis at the longitudinal end of the signal output pad to define a longitudinal end of the electrode, the traverse axis being orthogonal to the longitudinal axis, and
wherein the signal collection surface is parallel to and offset from the longitudinal axis and offset from the first and second surfaces of the signal output pad;
**characterized in that** the signal output terminal is attached to the signal output pad and extends along a transverse direction which is orthogonal to the longitudinal axis and has a signal output surface which surrounds the forward-end surface of the signal output pad; wherein the signal output terminal is configured as an elongate C-shaped clamp, the C-shaped clamp having a main body which is a hollow metal bar defining an internal surface and an external surface surrounding the internal surface; and wherein the internal surface is in compressive physical abutment with the signal output pad and the external surface is the signal output surface.

2. The electrode of claim 1, wherein the electrode is elongate and extends along the longitudinal axis, and the signal collection portion, the bridging portion and the signal output portion are distributed along the longitudinal axis in series;
wherein the signal output surface is an elongate surface extending along a transverse direction which is orthogonal to the longitudinal axis of the electrode;
wherein the signal output terminal is in compressive contact with the signal output end of the signal output portion.

3. The electrode according to any preceding claim, wherein the signal collection portion and the bridging portion are enclosed by an electrically insulating elastomeric sleeve except at the signal collection surface, and wherein the elastomeric sleeve is formed of a non-conductive elastomeric polymer and is formed as a water-tight enclosure.

4. The electrode according to any preceding claim, wherein the electrode has a maximum resistance of less than 600 ohm, measured between the signal output terminal and the signal collection surface which is a body contact surface.

5. The electrode according to any preceding claim, wherein the signal output terminal extends away from a forward end of the signal output pad, the forward end of the signal output pad being a longitudinal end most distal from the signal collection portion.

6. The electrode according to any preceding claim, wherein the signal output terminal comprises a signal output surface, wherein the signal output surface comprising a first portion which flares away from the longitudinal axis in an acute angle on extending away from the signal output end of the signal output pad to form an enlarged head portion on a longitudinal free end of the electrode; and/or wherein the signal output surface comprising a second portion which flares away from the longitudinal axis in an acute angle on extending away from the signal output end of the signal output pad to form an enlarged head portion on a longitudinal free end of the electrode.

7. The electrode according to claim 6, wherein the signal output surface comprises a third portion, the third portion comprising a forward-facing convex surface interconnecting the first portion and the second portion; and wherein the forward-facing convex surface extends along a transverse direction to form an enlarged head portion on the longitudinal free end of the electrode; and/or wherein the signal output surface extends in a transverse direction for the width of the signal output end.

8. The electrode according to any preceding claim, wherein the signal output terminal comprises a conductive main body and an open channel formed through the main body, wherein the main body is elongate and has a main axis which is orthogonal to the longitudinal axis.

9. The electrode according to any preceding claim, wherein the signal output terminal comprises an elongate internal surface which defines an open channel and an external surface surrounding the internal surface, and wherein the internal surface is in compressive physical abutment with the signal output pad to facilitate electrical contact between the signal output pad and the signal output terminal.

10. The electrode according to claim 9, wherein the internal surface is a signal input surface of the signal output terminal and the external surface is a signal output surface of the signal output terminal.

11. The electrode according to any preceding claim, wherein the signal output terminal is configured as a conductive anchor for physical retention by and electrical contact with an electronic signal receiver.

12. A performance monitoring apparatus comprising an electrode or a plurality of electrodes according to any of the preceding claim and an electronic device connected to the electrode or the plurality of electrodes, wherein the electrode is configured for collecting ECG signals and the electronic device is configured for collecting and processing ECG signals.

13. The apparatus of claim 12, wherein the electronic signal collection device comprises a main housing, a receptacle or a plurality of receptacles on the main housing for receiving a signal output terminal or a corresponding plurality of signal output terminals of the electrodes and electronic circuitry for processing ECG signals collected by the electrode.

14. The apparatus of claim 13, wherein the receptacle is configured for snap reception of a signal input terminal and comprises an entry aperture to permit entry of the signal input terminal and a slit which extends from the entry to permit movement of the signal output pad along an axial direction of the receptacle; or wherein the receptacle comprises a signal input surface, and wherein the signal input surface of the electronic device and the signal output surface of the electrode are in compressive frictional contact.

15. The apparatus of claim 13, wherein the receptacle and the signal output terminal are mechanically configured such that the electrode is mechanically retained by the electronic device when the signal output terminal is inserted into a channel of the receptacle in a direction of insertion and the electrode is removable from the receptacle by sliding in a direction opposite to the direction of insertion upon overcoming a retention force.

## Patentansprüche

1. Eine Elektrode zur Erfassung physiologischer Signale, beinhaltend einen Signalerfassungsabschnitt (122, 222), einen Signalausgangsabschnitt (124, 224), der ein Signalausgangsende aufweist, und einen Brückenabschnitt, der den Signalerfassungsabschnitt und den Signalausgangsabschnitt miteinander verbindet;
wobei der Signalerfassungsabschnitt (122, 222), der Brückenabschnitt und der Signalausgangsabschnitt (124, 224) entlang einer Längsachse in Reihe verteilt sind und sich der Signalausgangsabschnitt auf einem Längsende der Längsachse befindet;
wobei der Signalerfassungsabschnitt eine Signalerfassungsoberfläche für die Erfassung physiologischer Signale von einem lebenden Körper durch Körperkontakt beinhaltet,
wobei der Signalausgangsabschnitt eine Signalausgangsplatte, die ein vorderes Längsende aufweist, und einen Signalausgangsanschluss (126, 226) an dem vorderen Längsende der Signalausgangsplatte beinhaltet;
wobei die Signalausgangsplatte eine erste Oberfläche, eine zweite Oberfläche und eine Umfangsoberfläche, die eine Endoberfläche umfasst, welche eine die erste Oberfläche und die zweite Oberfläche miteinander verbindende vordere Endoberfläche ist, aufweist, wobei der Signalausgangsanschluss sowohl mit der ersten Oberfläche als auch mit der zweiten Oberfläche im physischen und elektrischen Kontakt steht und eine Signalausgangsoberfläche aufweist, die konfiguriert ist, um durch den Signalerfassungsabschnitt erfasste Signale auszugeben, und die eine äußere Oberfläche ist, welche zum Herstellen einer elektrischen Verbindung freiliegt und sich um eine Querachse an dem Längsende der Signalausgangsplatte erstreckt, um ein Längsende der Elektrode zu definieren, wobei die Querachse zu der Längsachse orthogonal ist, und
wobei die Signalerfassungsoberfläche zu der Längsachse parallel und von ihr versetzt ist und von der ersten und der zweiten Oberfläche der Signalausgangsplatte versetzt ist;
**dadurch gekennzeichnet, dass** der Signalausgangsanschluss an der Signalausgangsplatte angebracht ist und sich entlang einer Querrichtung, die zu der Längsachse orthogonal ist, erstreckt und eine Signalausgangsoberfläche aufweist, die die vordere Endoberfläche der Signalausgangsplatte umgibt; wobei der Signalausgangsanschluss als eine längliche C-förmige Klemme konfiguriert ist, wobei die C-förmige Klemme einen Hauptkörper aufweist, der ein hohler Metallstab ist, welcher eine interne Oberfläche und eine die interne Oberfläche umgebende externe Oberfläche definiert; und wobei die interne Oberfläche an die Signalausgangsplatte physisch pressend anstößt und die externe Oberfläche die Signalausgangsoberfläche ist.

2. Elektrode gemäß Anspruch 1, wobei die Elektrode länglich ist und sich entlang der Längsachse erstreckt, und wobei der Signalerfassungsabschnitt, der Brückenabschnitt und der Signalausgangsabschnitt entlang der Längsachse in Reihe verteilt sind; wobei die Signalausgangsoberfläche eine längliche Oberfläche ist, die sich entlang einer Querrichtung, welche zu der Längsachse der Elektrode orthogonal ist, erstreckt; wobei der Signalausgangsanschluss mit dem Signalausgangsende des Signalausgangsabschnitts in Presskontakt steht.

3. Elektrode gemäß einem der vorhergehenden Ansprüche, wobei der Signalerfassungsabschnitt und der Brückenabschnitt außer an der Signalerfassungsoberfläche von einer elektrisch isolierenden elastomeren Hülle eingeschlossen sind, und wobei die elastomere Hülle aus einem nicht leitenden elastomeren Polymer gebildet ist und als eine wasserdichte Umhüllung gebildet ist.

4. Elektrode gemäß einem der vorhergehenden Ansprüche, wobei die Elektrode einen maximalen Widerstand von weniger als 600 Ohm aufweist, gemessen zwischen dem Signalausgangsanschluss und der Signalerfassungsoberfläche, die eine Körperkontaktoberfläche ist.

5. Elektrode gemäß einem der vorhergehenden Ansprüche, wobei sich der Signalausgangsanschluss von einem vorderen Ende der Signalausgangsplatte weg erstreckt, wobei das vordere Ende der Signalausgangsplatte ein am weitesten distal von dem Signalerfassungsabschnitt liegendes Längsende ist.

6. Elektrode gemäß einem der vorhergehenden Ansprüche, wobei der Signalausgangsanschluss eine Signalausgangsoberfläche beinhaltet, wobei die Signalausgangsoberfläche einen ersten Abschnitt beinhaltet, der sich von der Längsachse weg in einem spitzen Winkel aufweitet, während er sich von dem Signalausgangsende der Signalausgangsplatte weg erstreckt, um einen vergrößerten Kopfabschnitt auf einem freien Längsende der Elektrode zu bilden; und/oder wobei die Signalausgangsoberfläche einen zweiten Abschnitt beinhaltet, der sich von der Längsachse weg in einem spitzen Winkel aufweitet, während er sich von dem Signalausgangsende der Signalausgangsplatte weg erstreckt, um einen vergrößerten Kopfabschnitt auf einem freien Längsende der Elektrode zu bilden.

7. Elektrode gemäß Anspruch 6, wobei die Signalausgangsoberfläche einen dritten Abschnitt beinhaltet, wobei der dritte Abschnitt eine vorwärts gerichtete konvexe Oberfläche beinhaltet, die den ersten Abschnitt und den zweiten Abschnitt miteinander verbindet; und wobei sich die vorwärts gerichtete konvexe Oberfläche entlang einer Querrichtung erstreckt, um einen vergrößerten Kopfabschnitt auf dem freien Längsende der Elektrode zu bilden; und/oder wobei sich die Signalausgangsoberfläche über die Breite des Signalausgangsendes in einer Querrichtung erstreckt.

8. Elektrode gemäß einem der vorhergehenden Ansprüche, wobei der Signalausgangsanschluss einen leitenden Hauptkörper und einen durch den Hauptkörper gebildeten offenen Kanal beinhaltet, wobei der Hauptkörper länglich ist und eine Hauptachse aufweist, welche zu der Längsachse orthogonal ist.

9. Elektrode gemäß einem der vorhergehenden Ansprüche, wobei der Signalausgangsanschluss eine längliche interne Oberfläche, die einen offenen Kanal definiert, und eine die interne Oberfläche umgebende externe Oberfläche beinhaltet, und wobei die interne Oberfläche an die Signalausgangsplatte physisch pressend anstößt, um den elektrischen Kontakt zwischen der Signalausgangsplatte und dem Signalausgangsanschluss zu ermöglichen.

10. Elektrode gemäß Anspruch 9, wobei die interne Oberfläche eine Signaleingangsoberfläche des Signalausgangsanschlusses ist und die externe Oberfläche eine Signalausgangsoberfläche des Signalausgangsanschlusses ist.

11. Elektrode gemäß einem der vorhergehenden Ansprüche, wobei der Signalausgangsanschluss als leitender Anker zur physischen Rückhaltung durch und für den elektrischen Kontakt mit einem elektronischen Signalempfänger konfiguriert ist.

12. Ein Leistungsüberwachungsgerät, das eine Elektrode oder eine Vielzahl von Elektroden gemäß einem der vorhergehenden Ansprüche und eine mit der Elektrode oder der Vielzahl von Elektroden verbundene elektronische Vorrichtung beinhaltet, wobei die Elektrode zum Erfassen von EKG-Signalen konfiguriert ist und die elektronische Vorrichtung zum Erfassen und Verarbeiten von EKG-Signalen konfiguriert ist.

13. Gerät gemäß Anspruch 12, wobei die elektronische Signalerfassungsvorrichtung ein Hauptgehäuse, ein Aufnahmefach oder eine Vielzahl von Aufnahmefächern auf dem Hauptgehäuse zum Aufnehmen eines Signalausgangsanschlusses oder einer entsprechenden Vielzahl von Signalausgangsanschlüssen der Elektroden und elektronische Schaltungen zum Verarbeiten von durch die Elektrode erfassten EKG-Signalen beinhaltet.

14. Gerät gemäß Anspruch 13, wobei das Aufnahmefach zur Schnappaufnahme eines Signaleingangsanschlusses konfiguriert ist und eine Eintrittsöffnung, um den Eintritt des Signaleingangsanschlusses zu gestatten, und einen Schlitz, der sich von dem Eintritt erstreckt, um die Bewegung der Signalausgangsplatte entlang einer axialen Richtung des Aufnahmefachs zu gestatten, beinhaltet; oder wobei das Aufnahmefach eine Signaleingangsoberfläche beinhaltet, und wobei die Signaleingangsoberfläche der elektronischen Vorrichtung und die Signalausgangsoberfläche der Elektrode in pressendem Reibkontakt stehen.

15. Gerät gemäß Anspruch 13, wobei das Aufnahmefach und der Signalausgangsanschluss mechanisch so konfiguriert sind, dass die Elektrode durch die elektronische Vorrichtung mechanisch zurückgehalten wird, wenn der Signalausgangsanschluss in einer Einführungsrichtung in einen Kanal des Aufnahmefachs eingeführt wird, und die Elektrode durch Verschieben in einer zu der Einführungsrichtung entgegengesetzten Richtung aus dem Aufnahmefach entnehmbar ist, wenn eine Rückhaltekraft überwunden wird.

## Revendications

1. Une électrode de collecte de signaux physiologiques comprenant une portion de collecte de signaux (122, 222), une portion de sortie de signaux (124, 224) présentant une extrémité de sortie de signaux, et une portion faisant le pont qui relie entre elles la portion de collecte de signaux et la portion de sortie de signaux ;
où la portion de collecte de signaux (122, 222), la portion faisant le pont et la portion de sortie de signaux (124, 224) sont distribuées en série le long d'un axe longitudinal et la portion de sortie de signaux est sur une extrémité longitudinale de l'axe longitudinal ; où la portion de collecte de signaux comprend une surface de collecte de signaux pour la collecte de signaux physiologiques provenant d'un corps vivant au contact du corps, où la portion de sortie de signaux comprend un plot de sortie de signaux présentant une extrémité longitudinale avant et une borne de sortie de signaux (126, 226) au niveau de l'extrémité longitudinale avant du plot de sortie de signaux ;
où le plot de sortie de signaux présente une première surface, une deuxième surface, et une surface périphérique qui inclut une surface d'extrémité qui est une surface d'extrémité avant reliant entre elles la première surface et la deuxième surface, où la borne de sortie de signaux est en contact physique et électrique à la fois avec la première surface et la deuxième surface, et présente une surface de sortie de signaux qui est configurée pour produire en sortie des signaux collectés par la portion de collecte de signaux et qui est une surface extérieure qui est apparente pour l'établissement d'une connexion électrique et s'étend autour d'un axe transverse au niveau de l'extrémité longitudinale du plot de sortie de signaux afin de définir une extrémité longitudinale de l'électrode, l'axe transverse étant orthogonal à l'axe longitudinal, et
où la surface de collecte de signaux est parallèle à et décalée vis-à-vis de l'axe longitudinal et décalée vis-à-vis des première et deuxième surfaces du plot de sortie de signaux ;
**caractérisée en ce que** la borne de sortie de signaux est attachée au plot de sortie de signaux et s'étend le long d'une direction transversale qui est orthogonale à l'axe longitudinal et présente une surface de sortie de signaux qui entoure la surface d'extrémité avant du plot de sortie de signaux ; où la borne de sortie de signaux est configurée telle une bride en forme de C allongée, la bride en forme de C présentant un corps principal qui est une barre en métal creuse définissant une surface interne et une surface externe entourant la surface interne ; et où la surface interne est en butée compressive physique avec le plot de sortie de signaux et la surface externe est la surface de sortie de signaux.

2. L'électrode de la revendication 1, où l'électrode est allongée et s'étend le long de l'axe longitudinal, et la portion de collecte de signaux, la portion faisant le pont et la portion de sortie de signaux sont distribuées le long de l'axe longitudinal en série ;
où la surface de sortie de signaux est une surface allongée s'étendant le long d'une direction transversale qui est orthogonale à l'axe longitudinal de l'électrode ;
où la borne de sortie de signaux est en contact compressif avec l'extrémité de sortie de signaux de la portion de sortie de signaux.

3. L'électrode selon n'importe quelle revendication précédente, où la portion de collecte de signaux et la portion faisant le pont sont encloses par un manchon élastomère électriquement isolant si ce n'est au niveau de la surface de collecte de signaux, et où le manchon élastomère est formé en un polymère élastomère non conducteur et est formé telle une enceinte étanche à l'eau.

4. L'électrode selon n'importe quelle revendication précédente, où l'électrode présente une résistance maximale inférieure à 600 ohms, mesurée entre la borne de sortie de signaux et la surface de collecte de signaux qui est une surface au contact du corps.

5. L'électrode selon n'importe quelle revendication précédente, où la borne de sortie de signaux s'étend à partir d'une extrémité avant du plot de sortie de signaux, l'extrémité avant du plot de sortie de signaux étant une extrémité longitudinale la plus distale vis-à-vis de la portion de collecte de signaux.

6. L'électrode selon n'importe quelle revendication précédente, où la borne de sortie de signaux comprend une surface de sortie de signaux, où la surface de sortie de signaux comprend une première portion qui s'évase en se distançant de l'axe longitudinal selon un angle aigu lorsqu'elle s'étend à partir de l'extrémité de sortie de signaux du plot de sortie de signaux afin de former une portion de tête élargie sur une extrémité longitudinale libre de l'électrode ; et/ou bien où la surface de sortie de signaux comprend une deuxième portion qui s'évase en se distançant de l'axe longitudinal selon un angle aigu lorsqu'elle s'étend à partir de l'extrémité de sortie de signaux du plot de sortie de signaux afin de former une portion de tête élargie sur une extrémité longitudinale libre de l'électrode.

7. L'électrode selon la revendication 6, où la surface de sortie de signaux comprend une troisième portion, la troisième portion comprenant une surface convexe tournée vers l'avant reliant entre elles la première portion et la deuxième portion ; et où la surface convexe tournée vers l'avant s'étend le long d'une direction transversale afin de former une portion de tête élargie sur l'extrémité longitudinale libre de l'électrode ; et/ou bien où la surface de sortie de signaux s'étend dans une direction transversale pour la largeur de l'extrémité de sortie de signaux.

8. L'électrode selon n'importe quelle revendication précédente, où la borne de sortie de signaux comprend un corps principal conducteur et un canal ouvert formé à travers le corps principal, où le corps principal est allongé et présente un axe principal qui est orthogonal à l'axe longitudinal.

9. L'électrode selon n'importe quelle revendication précédente, où la borne de sortie de signaux comprend une surface interne allongée qui définit un canal ouvert et une surface externe entourant la surface interne, et où la surface interne est en butée compressive physique avec le plot de sortie de signaux afin de faciliter le contact électrique entre le plot de sortie de signaux et la borne de sortie de signaux.

10. L'électrode selon la revendication 9, où la surface interne est une surface d'entrée de signaux de la borne de sortie de signaux et la surface externe est une surface de sortie de signaux de la borne de sortie de signaux.

11. L'électrode selon n'importe quelle revendication précédente, où la borne de sortie de signaux est configurée telle une ancre conductrice pour la rétention physique par et le contact électrique avec un récepteur de signaux électroniques.

12. Un appareil de surveillance de performance comprenant une électrode ou une pluralité d'électrodes selon n'importe lesquelles des revendications précédentes et un dispositif électronique relié à l'électrode ou à la pluralité d'électrodes, où l'électrode est configurée pour collecter des signaux ECG et le dispositif électronique est configuré pour collecter et traiter des signaux ECG.

13. L'appareil de la revendication 12, où le dispositif de collecte de signaux électroniques comprend un boîtier principal, un réceptacle ou une pluralité de réceptacles sur le boîtier principal pour recevoir une borne de sortie de signaux ou une pluralité correspondante de bornes de sortie de signaux des électrodes et une circuiterie électronique pour traiter des signaux ECG collectés par l'électrode.

14. L'appareil de la revendication 13, où le réceptacle est configuré pour la réception par enclenchement d'une borne d'entrée de signaux et comprend une ouverture d'entrée afin de permettre l'entrée de la borne d'entrée de signaux et une fente qui s'étend depuis l'entrée afin de permettre un déplacement du plot de sortie de signaux le long d'une direction axiale du réceptacle ; ou bien où le réceptacle comprend une surface d'entrée de signaux, et où la surface d'entrée de signaux du dispositif électronique et la surface de sortie de signaux de l'électrode sont en contact compressif par friction.

15. L'appareil de la revendication 13, où le réceptacle et la borne de sortie de signaux sont configurés mécaniquement de telle sorte que l'électrode est retenue mécaniquement par le dispositif électronique lorsque la borne de sortie de signaux est insérée dans un canal du réceptacle dans une direction d'insertion et que l'électrode peut être retirée du réceptacle par coulissement dans une direction opposée à la direction d'insertion dès qu'il est franchi une force de rétention.
